# EUROPEAN PATENT APPLICATION

(11) **EP 3 974 516 A1**
(43) Date of publication of application: **30.03.2022**
(21) Application number: 20386047.3
(22) Date of filing: 24.09.2020
(51) Int. Cl.: C12M 3/00, C12M 1/12, A61M 1/00, A61M 27/00, C12M 1/42

(54) **PHYSIOLOGICAL SIMULATION DEVICE**

(71) Applicant: T.J. Smith and Nephew, Limited, Hull HU3 2BN (GB)
(72) Inventor: Aspioti, Maria, Hull HU3 2BN (GB); Brownhill, Varuni Rachindra, Hull HU3 2BN (GB); Hardman, Matthew James, Hull HU6 7RX (GB); Roberts, Elizabeth R., Hull HU6 7RX (GB); France, Louise Amy, Hull HU6 7RX (GB)
(74) Representative: Appleyard Lees IP LLP

(57) **Abstract**

A physiological simulation device comprising a permeable tissue mimicking construct, wherein a first side of the construct is exposed to a tissue culture media and a second side of the construct is operable to have tissue arranged thereon, wherein the tissue mimicking construct is arranged and operable to allow the flow of tissue culture media from the first side of the construct to the second side of the construct to thereby contact living tissue arranged thereon.

## Description

### FIELD

Embodiments of the present invention relate to a physiological simulation device useful in observing biological activity and the effects of therapeutic treatment, in particular wound care treatments, of large-scale *ex vivo* culture systems. The device and methods can incorporate or implement any combination of the features described below.

### BACKGROUND

Elucidating the cellular and molecular mode of action resulting from therapeutic activity, such as wound care treatment, on cellular systems is highly beneficial in aiding the development of these treatments and their methods of use.

Negative pressure wound therapy is a widely used wound care treatment for chronic, non-healing wounds. Treatment of chronic wounds consumes a great deal of resources globally, therefore advancement in negative pressure wound therapy and its methods of use is imperative. However, previous experimental set ups to systematically evaluate the cellular and molecular effects of negative pressure treatment are limited. Therefore, there is a requirement for technology to enable direct monitoring and assessment of biological activity in large culture systems being treated by negative pressure wound therapy.

*In vitro* cell work requires cumbersome chamber set-ups to deliver negative pressure and do not always represent correct clinical delivery of negative pressure. *Ex vivo* wound models are more ethical and less risky than *in vivo* studies and invasive clinical studies.

However, the experimental setup in prior art *ex vivo* tissue models for assessing negative pressure wound therapy presents high variability depending on the test method and outcome measures required. Tissue biopsies are typically used as models to evaluate different therapeutic interventions. However, due to the limitations of tissue biopsies in size and shape, they do not represent ideal models to incorporate larger systems and are thereby incompatible with the application of negative pressure wound therapy devices.

Alternatively, the biological effects of negative pressure wound therapy can be monitored *ex vivo* on cell cultures. Typically, in these studies cell cultures are placed in a vacuum chamber connected to a vacuum pump and remain under pressure for a period of time before being removed and studied. However, these studies present the effects of changing pressure only on isolated cell cultures, posttreatment.

Therefore, there is a requirement for a physiological simulation device which allows for the monitoring and evaluation of biological efficacy of negative pressure wound therapy in a preclinical system using a large tissue explant.

The present invention relates to a viable large-scale *ex vivo* tissue culture model that is suitable for testing wound care devices. The present invention allows the user to monitor and assess biological cellular activities before, during and after wound care treatments of a large-scale *ex vivo* culture system. The *ex vivo* physiological simulation device provides a system that can be used with clinical devices and provides an ideal way to study cellular and tissue wound healing benefits of negative pressure wound therapy. The present invention further allows for the evaluation of biological efficacy of various concepts and prototypes for negative pressure wound therapy.

### SUMMARY

Embodiments of the present disclosure are directed to a physiological simulation device and methods of use in monitoring biological activity and assessing therapeutic activity, for example negative pressure wound therapy.

According to a first embodiment of the invention there is provided a physiological simulation device comprising a permeable tissue mimicking construct, wherein a first side of the construct is exposed to a tissue culture media and a second side of the construct is operable to have tissue arranged thereon, wherein the tissue mimicking construct is arranged and operable to allow the flow of tissue culture media from the first side of the construct to the second side of the construct to thereby contact living tissue arranged thereon.

The tissue arranged on the physiological simulation device is preferably living tissue, such as skin tissue. The living tissue, which may be skin tissue, preferably comprises a wound area.

The physiological simulation device is arranged and operable to support and maintain living tissue, such as skin tissue, to allow for monitoring and assessment of biological cellular activities before, during and after wound care treatments. This arrangement suitably allows for the advancement of wound care methods and technology.

The permeable tissue mimicking construct of the physiological simulation device may comprise a skin mimicking tissue. The permeable tissue mimicking construct may have a thickness of between 1 and 5 cm to represent dermis of varying dimensions and rigidity.

The permeable tissue mimicking construct of the physiological simulation device may comprise silicone, latex, rubber, or other polymer-based materials or a combination thereof.

The permeable tissue mimicking construct of the physiological simulation device may comprise one or more wound area. The simulated wound area may comprise a depression, cavity or recess. The depression, cavity or recess may have any suitable size, such as, for example, between 1 cm² and 20 cm². The depression, cavity or recess may have any suitable shape, such as circular, oval, elliptical, etc. The depression, cavity or recess may have any suitable depth, such as, for example between 1 mm and 3 cm.

The permeable tissue mimicking construct of the physiological simulation device may comprise one or more channels therethrough, the channels being arranged and operable to allow the tissue culture media to flow from the first side of the construct to the second side of the construct. The channels may be arranged in a uniform manner. The channels may have an internal diameter of between 1 and 10 mm, such as between 2 and 8 mm, or between 3 and 7 mm. The channels may have an internal diameter of between about 4 and 5 mm. The channels may comprise at least 1% of the volume of the tissue mimicking construct, such as 2 to 8%, or even 4 to 6%.

The permeable tissue mimicking construct of the physiological simulation device may comprise a layer of wicking material. One or more of the channels may comprise wicking material, which may extend substantially therethrough, and may be operable, in use, to aid capillary action of the tissue culture media to flow from the first side of the construct to the second side of the construct. The wicking material may be arranged within the channels or arranged thereon. The wicking material may comprise materials which enable a flow rate of the tissue culture media from the first side of the tissue mimicking construct to the second side of the construct of at least between 3.0 and 3.5 mm min⁻¹. The wicking material may be arranged to form a porous network within the channels. Non-limiting examples of suitable wicking materials include paper-based materials, microfluidic materials or a combination thereof. Non-limiting examples of paper based wicking materials include chromatography paper, absorbent cellulose pads, tissue or a combination thereof. Non-limiting examples of microfluidic wicking materials include hydrogels, biodegradable materials or a combination thereof.

The permeable tissue mimicking construct of the physiological simulation device may be arranged within a base device, which base device may be operable to contain the tissue culture media.

The tissue culture media of the physiological simulation device may comprise nutrient supplementation. The tissue culture media may comprise foetal bovine serum, antibiotic solution, antimycotic solution, amphotericin B or a combination thereof.

According to the second aspect of the invention there is provided a negative pressure wound therapy simulation device, said negative pressure wound therapy simulation device comprises:
i. a physiological simulation device of the first embodiment;
ii. a negative pressure wound therapy dressing arranged over at least a portion of the second side of the permeable tissue mimicking construct of the physiological simulation device, to thereby allow negative pressure to be applied to tissue arranged thereon, in use.

According to a third aspect of the invention there is provided a method of applying negative pressure wound therapy to *ex vivo* tissue, said method of applying negative pressure wound therapy to ex *vivo* tissue comprises the steps:
i. arranging tissue, such as skin tissue, on a second side of a tissue mimicking construct of a physiological simulation device of the first embodiment;
ii. arranging a negative pressure wound therapy dressing over at least a portion of the tissue; and
iii. applying negative pressure wound therapy to the tissue, via the negative pressure wound therapy dressing.

According to a fourth aspect of the invention there is provided a method of monitoring the biological processes of wound healing, said method of monitoring the biological processes of wound healing comprises the steps:
i. arranging tissue having a wounded portion, such as skin tissue having a wounded portion, on a second side of a tissue mimicking construct of a physiological simulation device of the first embodiment;
ii. arranging a negative pressure wound therapy dressing over the wounded portion of the tissue;
iii. applying negative pressure wound therapy to the wounded portion of the tissue via the negative pressure wound therapy dressing; and
iv. monitoring the wounded portion of the tissue.

According to a fifth aspect of the invention there is provided a method of applying negative pressure wound therapy to *ex vivo* colonised tissue, said method of applying negative pressure wound therapy to *ex vivo* colonised tissue comprises the steps:
i. colonising tissue, such as skin tissue, with pathogens and/or bacteria;
ii. arranging the colonised tissue on a second side of a tissue mimicking construct of a physiological simulation device of the first embodiment;
iii. arranging a negative pressure wound therapy dressing over at least a portion of the colonised tissue; and
iv. applying negative pressure wound therapy to the colonised tissue, via the negative pressure wound therapy dressing.

According to a sixth aspect of the invention there is provided a method of monitoring the biological processes of infection and wound healing, said method of monitoring the biological processes of infection and wound healing comprises the steps:
i. colonising tissue having a wounded portion, such as skin tissue having a wounded portion, with pathogens and/or bacteria;
ii. arranging the colonised tissue having a wounded portion on a second side of a tissue mimicking construct of a physiological simulation device of the first embodiment;
iii. arranging a negative pressure wound therapy dressing over the wounded portion of the colonised tissue;
iv. applying negative pressure wound therapy to the wounded portion of the colonised tissue via the negative pressure wound therapy dressing; and
v. monitoring the wounded portion of the colonised tissue.

### BRIEF DESCRIPTION OF DRAWINGS

Embodiments of the present disclosure will now be described hereinafter, by way of example only, with reference to the accompanying drawings in which:
Figure 1 shows a plan view of a physiological simulation device;
Figure 2 shows a cross sectional view of a physiological simulation device;
Figure 3 shows a cross sectional view of a negative pressure wound therapy simulation device in combination with the physiological simulation device; and
Figure 4 shows an exploded view of a negative pressure wound therapy simulation device in combination with the physiological simulation device.

### DESCRIPTION OF EMBODIMENTS

Some embodiments disclosed herein relate to wound therapy on human or animal tissue. Therefore, any reference to a wound herein can refer to a wound on human or animal tissue.

As used herein the expression "wound" or "wounded portion" may include any injury to living tissue and may be caused by a cut, laceration, burn, pressure, blow, or other impact, typically one in which the tissue is cut or broken. The wound may include injury to the epidermis, dermis or hypodermis or a combination thereof.

As used herein the expression "wound area" may include any depression, cavity or recess made in a synthetic tissue mimicking material.

The wound therapies that may be utilized in conjunction with the disclosed technology include any known wound therapies in the art. The technology is applicable to negative pressure wound therapy treatment.

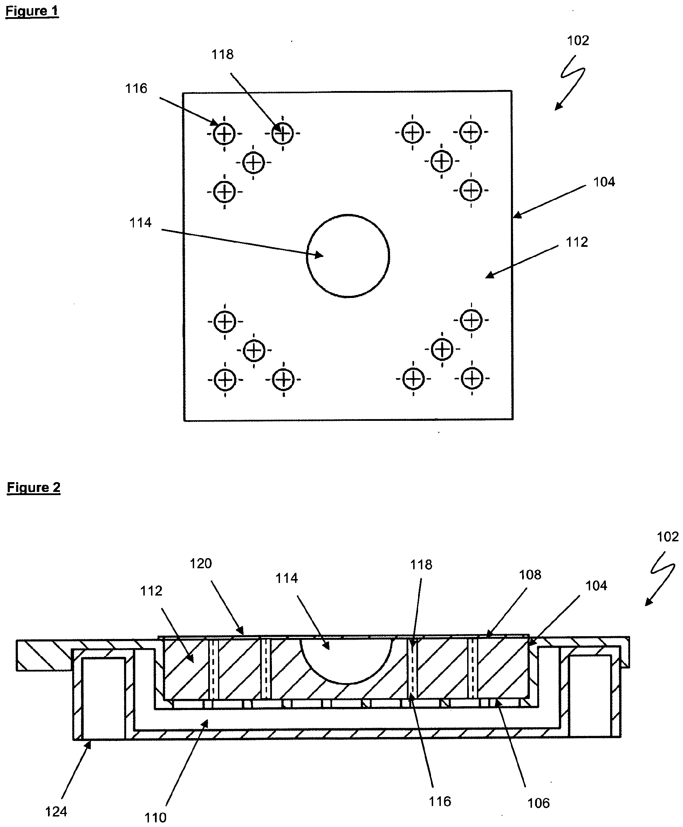

Referring first to Figures 1 and 2, there is shown a physiological simulation device 102 according to the invention. A permeable tissue mimicking construct 104 is provided, wherein a first side of the construct 106 is exposed to a tissue culture media 110 and a second side of the construct 108 is operable to have tissue 120 arranged thereon, wherein the tissue mimicking construct 104 is arranged and operable to allow the flow of tissue culture media 110 from the first side of the construct 106 to the second side of the construct 108 to thereby contact and supply the living tissue 120 arranged thereon.

The permeable tissue mimicking construct 104 comprises a skin mimicking material 112. The skin mimicking material 112 comprises a tissue-compatible material that possesses bio-mechanical properties similar to that of skin tissue. Preferably, the skin mimicking material 112 has a fracture strength of at least between 1 and 2 N/mm², a fracture strain of at least between 300 and 400% and a tear resistance of at least between 10 and20 N/mm.

Preferably, the permeable tissue mimicking construct 104 is arranged to have a thickness of at least between 1 and 5 cm to mimic clinically relevant wound area size and shape while maintaining a desired flow rate of the tissue culture media 110 from the first side of the tissue mimicking construct 106 to the second side of the tissue mimicking construct 108 of at least between 3.0 and 3.5 mm min⁻¹.

The permeable tissue mimicking construct 104 may comprise, without limitation, silicone, latex, rubber, other polymer-based materials or a combination thereof.

Figures 1 and 2 also show a wound area 114 in the permeable tissue mimicking construct 104. The wound area 114 comprises a depression, cavity or recess. The wound area 114 may be arranged in any suitable shape or dimension to mimic a chronic wound.

The wound area 114 may be provided by a surgical blade or any other suitable instrument. The wound area may be any suitable size or shape. Preferably, the wound area 114 has a diameter of between 3 and 9 cm. By "diameter" in this context, it should be understood that the wound area is not necessarily circular, but could be any suitable shape. "Diameter" in this context, therefore, refers to the longest distance between generally opposite edges of the wound area. Preferably, the wound area has a depth of between 0.5 and 4.5 cm. By wound area "depth" in this context it is meant the amount to which the wound area extends into a surface of the tissue mimicking construct 104. The wound area 114 in the permeable tissue mimicking construct 104 is arranged to align with the wound of the living tissue 120.

Channels 116 in the permeable tissue mimicking construct 104 are also provided in Figures 1 and 2. The channels 116 are arranged and operable to allow the tissue culture media 110 to flow from the first side of the permeable tissue mimicking construct 106 to the second side of the tissue mimicking construct 108 via capillary action. The channels 116 may be in a uniform arrangement within in the permeable tissue mimicking construct 104 around the wound area 114 present in numbers to maximise the supply of tissue culture media 110 to the living tissue 120 while retaining the mechanistic properties of the permeable tissue mimicking construct 104. The channels 116 may have a diameter of at least between 1 and 10 mm to allow a flow rate of the tissue culture media 110 from the first side of the permeable tissue mimicking construct 106 to the second side of the tissue mimicking construct 108 of at least between 3.0 and 3.5 mm min⁻¹.

In another embodiment, the channels 116 may be in a uniform arrangement within in the permeable tissue mimicking construct 104 around the wound area 114 present in numbers to maximise the supply of tissue culture media 110 to the living tissue 120 while retaining the mechanistic properties of the permeable tissue mimicking construct 104. The channels 116 may have a diameter of at least between1 and10 mm to allow a flow rate of the tissue culture media 110 from the first side of the permeable tissue mimicking construct 106 to the second side of the tissue mimicking construct 108 of at least between 3.0 and 3.5 mm min⁻¹. The channels 116 may also be arranged within the wound area 114. The channels 116 within the wound area 114 are arranged to be supplied with tissue culture media 110 though a peristaltic pump. The flow rate at which the tissue culture media 110 is supplied to the wound area 114 is set mimic wound exudate being produced by the wound area 114. The flow rate is controlled to mimic low, moderate and high exuding wounds. Preferably the flow rate of the tissue culture media 110 to the wound area 114 is at least between 0.6g of liquid exudate per cm² of wound area per 24 hours and 2.8g of liquid exudate per cm² of wound area per 24 hours. The flow rate may be determined through absorption of tissue culture media and evaporation at the tissue surface, with evaporation controlling the flux in the system and is defined through evaporimeter measurements. A 10 x 10 cm tissue explant consumes at least between 10 and 20 ml of tissue culture media per 24 hours.

Optionally, the channels 116 may further comprise a layer of wicking material 118. One or more of the channels 116 may comprise a layer of wicking material 118 to aid capillary action of the tissue culture media 110 to flow from the first side of the permeable tissue mimicking construct 106 to the second side of the tissue mimicking construct 108 to supply to the living tissue 120 arranged thereon. The wicking material 118 may be arranged within the channels 116 or arranged thereon. The wicking material 118 may also be arranged to form a porous network within the channels 116. The wicking material 118 may comprise materials which enable a flow rate of the tissue culture media 110 from the first side of the permeable tissue mimicking construct 106 to the second side of the tissue mimicking construct 108 of at least between 3.0 and 3.5 mm min⁻¹ Non-limiting examples of suitable wicking materials include paper-based materials, microfluidic materials or a combination thereof. Non-limiting examples of paper based wicking materials include chromatography paper, absorbent cellulose pads, tissue or a combination thereof. Non-limiting examples of microfluidic wicking materials include hydrogels, biodegradable materials or a combination thereof

The permeable tissue mimicking construct 104 of the physiological simulation device 102 may be arranged within a base 124 containing tissue culture media 110. The base 124 may comprise any suitable container such as a cassette, petri dish, etc.

The living tissue 120, such as skin tissue, arranged on the second side of the permeable tissue mimicking construct 108 is supported and maintained by the controlled supply of tissue culture media 110 from the first side of the construct 106 to the second side of the construct 108 via channels 116 arranged therethrough.

The tissue culture media 110 may comprise nutrient supplementation. The tissue culture media 110 operates to supply the living tissue 120 with the nutrients required to enable normal function of the living tissue 120 to allow for *ex vivo* wound healing to be assessed. The tissue culture media 110 may comprise foetal bovine serum, antibiotic solution, antimycotic solution, amphotericin B or a combination thereof. The tissue culture media 110 may be provided a commercial standard, a non-limiting example may include Dulbecco's Modified Eagle Media (DMEM) containing 10% foetal bovine serum, 1% penicillin-streptomycin solution and 1% amphotericin B.

For the avoidance of doubt, the flow rate of the tissue culture media 110 from the first side of the permeable tissue mimicking construct 106 to the second side of the tissue mimicking construct 108 is dependent upon the thickness of the permeable tissue mimicking construct 104, diameter of channels 116, presence and type of wicking material 118 and viscosity of tissue culture media 110. Preferably these parameters of the physiological simulation device 102 are arranged to permit a flow rate of at least between 3.0 and 3.5 mm min⁻¹. Fluid flow is determined through absorption of tissue culture media 110 and evaporation at the skin surface, with evaporation controlling the flux in the system and is defined through evaporimeter measurements. A 10 x 10 cm tissue explant consumes at least between 10 and 20 ml of tissue culture media per 24 hours.

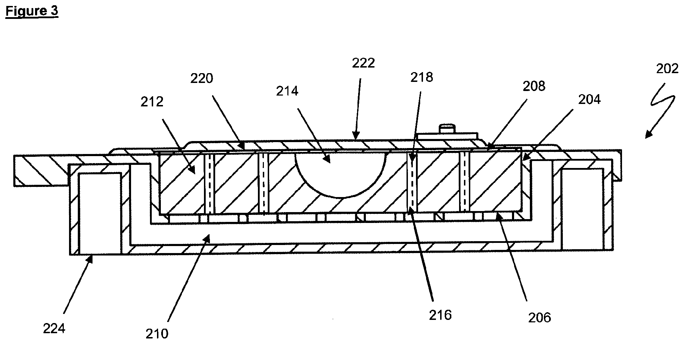

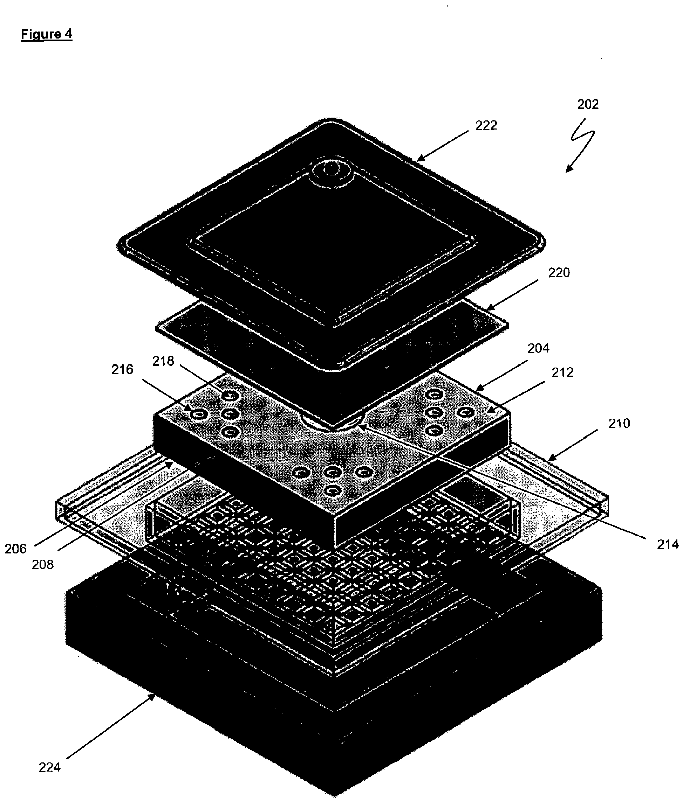

Referring to Figures 3 and 4, there is shown a negative pressure wound therapy simulation device 202 according to the invention. The negative pressure wound therapy simulation device 202 comprises the physiological simulation device 102 of the present invention with a negative pressure wound therapy dressing 222 arranged over at least a portion of the second side of the permeable tissue mimicking construct 208 of the physiological simulation device 102, to thereby allow negative pressure to be applied to tissue 220 arranged thereon, in use.

A third aspect of the invention relates to a method of applying negative pressure wound therapy to *ex vivo* tissue comprising the steps:
i. arranging tissue 220, such as skin tissue, on a second side of a tissue mimicking construct 208 of the physiological simulation device 102;
ii. arranging a negative pressure wound therapy dressing 222 over at least a portion of the tissue 220; and
iii. applying negative pressure wound therapy to the tissue 220, via the negative pressure wound therapy dressing 222.

The wound therapies that may be utilized in conjunction with the disclosed technology include any known wound therapies in the art. The wound therapies that may be applied include, but are not limited to, negative pressure wound therapy.

The dimensions of the components of the physiological simulation device 102 are arranged to maximise use with clinical devices and prototypes for negative pressure therapy. Preferably the physiological simulation device 102 is arranged to be operable to have at least a 10 x 10 cm tissue explant arranged thereon.

A fourth aspect of the invention relates to a method of monitoring the biological processes of wound healing comprising the steps:
i. arranging tissue having a wounded portion 220, such as skin tissue having a wounded portion, on a second side of a tissue mimicking construct 208 of the physiological simulation device 102;
ii. arranging a negative pressure wound therapy dressing 222 over the wounded portion of the tissue 220;
iii. applying negative pressure wound therapy to the wounded portion of the tissue 220 via the negative pressure wound therapy dressing 222; and
iv. monitoring the wounded portion of the tissue 220.

A fifth aspect of the invention relates to a method of applying negative pressure wound therapy to *ex vivo* colonised tissue, said method of applying negative pressure wound therapy to *ex vivo* colonised tissue comprises the steps:
i. colonising tissue 220, such as skin tissue, with pathogens and/or bacteria;
ii. arranging the colonised tissue 220 on a second side of a tissue mimicking construct 280 of the physiological simulation device 102 of;
iii. arranging a negative pressure wound therapy dressing 222 over at least a portion of the colonised tissue 220; and
iv. applying negative pressure wound therapy to the colonised tissue 220, via the negative pressure wound therapy dressing 222.

A sixth aspect of the invention relates to a method of monitoring the biological processes of infection and wound healing, said method of monitoring the biological processes of infection and wound healing comprises the steps:
i. colonising tissue having a wounded portion 220, such as skin tissue having a wounded portion, with pathogens and/or bacteria;
ii. arranging the colonised tissue having a wounded portion 220 on a second side of a tissue mimicking construct 208 of the physiological simulation device 102;
iii. arranging a negative pressure wound therapy dressing 222 over the wounded portion of the colonised tissue 220;
iv. applying negative pressure wound therapy to the wounded portion of the colonised tissue 220 via the negative pressure wound therapy dressing 222; and
v. monitoring the wounded portion of the colonised tissue 220.

The methods of monitoring biological processes of wound healing that may be utilized in conjunction with the disclosed technology include any known methods in the art.

Monitoring biological cellular activities of wound healing may be carried out before, during and/or after wound care treatment. Typically, cellular and molecular activity is monitored post treatment.

The biological processes of wound healing and methods of monitoring include, but are not limited to, epidermal-dermal junction damage and stratum corneum thickness by H&E staining, epidermal apoptosis by TUNEL staining, skin humidity by TEWL measurement, epidermal cell proliferation by Ki67 staining, skin surface lipids by Oil Red O staining, wound closure by whole mount K14 staining, assessment of extracellular matrix by picrosirius red staining any other tissue morphology measures possible.

Online measurements and live phase monitoring that may be carried out during treatment include, but are not limited to, using sensors or indicator dyes.

Further methods of monitoring the biological processes of wound healing include, but are not limited to, assessment of tissue culture media parameters, such as pH, oxygenation status and the presence of exocrine molecules.

With the embodiments of the present disclosure, a physiological simulation device 102 is provided that is useful for supporting, maintaining and observing the cellular activity of a living tissue system in response to wound therapy treatment, further aiding the future development of negative pressure wound therapy.

### Test Methods

**Fracture Strength:** The force required to cause a fracture in a length of material. The ability of a material to resist failure and is designated specifically according to the mode of applied loading, such as tensile, compressive, or bending.

**Fracture Strain:** The percentage elongation from the initial length, and length at elongation post fracture of the material. The ratio between changed length and initial length after breakage of the test specimen.

**Tear Resistance:** The force required to propagate a tear through a length of material. A measure of how well a material can withstand the effects of tearing.

### Methods of monitoring biological processes

Staining methods to assess tissue morphology and function include, but are not limited to, hematoxylin and eosin (H&E) staining, terminal deoxynucleotidyl transferase dUTP nick end labelling (TUNEL) staining, protein staining including Ki67 staining and whole mount K14 staining, Oil Red O staining and picrosirius red staining.

Measurements to assess tissue morphology and function may also include transepidermal water loss (TEWL) measurement and any other tissue morphology measures possible.

All methods and measurements carried out are known in the art and use standard procedure, equipment and commercially available chemicals.

Although the present disclosure includes certain embodiments, examples and applications, it will be understood by those skilled in the art that the present disclosure extends beyond the specifically disclosed embodiments to other alternative embodiments or uses and obvious modifications and equivalents thereof, including embodiments which do not provide all of the features and advantages set forth herein. Accordingly, the scope of the present disclosure is not intended to be limited by the specific disclosures of preferred embodiments herein, and may be defined by claims as presented herein or as presented in the future.

Conditional language, such as "can," "could," "might," or "may," unless specifically stated otherwise, or otherwise understood within the context as used, is generally intended to convey that certain embodiments include, while other embodiments do not include, certain features, elements, or steps. Thus, such conditional language is not generally intended to imply that features, elements, or steps are in any way required for one or more embodiments or that one or more embodiments necessarily include logic for deciding, with or without user input or prompting, whether these features, elements, or steps are included or are to be performed in any particular embodiment. The terms "comprising," "including," "having," and the like are synonymous and are used inclusively, in an openended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Further, the term "each," as used herein, in addition to having its ordinary meaning, can mean any subset of a set of elements to which the term "each" is applied.

Throughout the description and claims of this specification, the singular encompasses the plural unless the context otherwise requires. In particular, where the indefinite article is used, the specification is to be understood as contemplating plurality as well as singularity, unless the context requires otherwise.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," "essentially" and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, or 0.1 degree.

Features, integers, characteristics, compounds, chemical moieties or groups described in conjunction with a particular aspect, embodiment or example of the present disclosure are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The scope of the present disclosure is not intended to be limited by the specific disclosures of preferred embodiments in this section or elsewhere in this specification, and may be defined by claims as presented in this section or elsewhere in this specification or as presented in the future. The language of the claims is to be interpreted broadly based on the language employed in the claims and not limited to the examples described in the present specification or during the prosecution of the application, which examples are to be construed as non-exclusive.

The reader's attention is directed to all papers and documents which are filed concurrently with or previous to this specification in connection with this application and which are open to public inspection with this specification, and the contents of all such papers and documents are incorporated herein by reference.

## Claims

1. A physiological simulation device comprising a permeable tissue mimicking construct, wherein a first side of the construct is exposed to a tissue culture media and a second side of the construct is operable to have tissue arranged thereon, wherein the tissue mimicking construct is arranged and operable to allow the flow of tissue culture media from the first side of the construct to the second side of the construct to thereby contact living tissue arranged thereon.

2. A device according to claim 1, wherein the permeable tissue mimicking construct comprises a skin mimicking material.

3. A device according to any proceeding claim, wherein the permeable tissue mimicking construct comprises silicone, rubber, latex, or other polymer-based materials or a combination thereof.

4. A device according to any proceeding claim, wherein the permeable tissue mimicking construct comprises one or more wound area.

5. A device according to claim 4, wherein the wound area comprises a depression, cavity or recess.

6. A device according to any proceeding claim, wherein the permeable tissue mimicking construct comprises one or more channels therethrough, the channels being arranged and operable to allow the tissue culture media to flow from the first side of the construct to the second side of the construct.

7. A device according to claim 6, wherein one or more of the channels may comprise a wicking material extending substantially therethrough to aid capillary action of the tissue culture media to flow from the first side of the construct to the second side of the construct.

8. A device according to any proceeding claim, wherein the tissue culture media comprises nutrient supplementation.

9. A device according to claim 8, wherein the tissue culture media comprises foetal bovine serum, antibiotic solution, antimycotic solution, amphotericin B or a combination thereof.

10. A negative pressure wound therapy simulation device comprising
i) A physiological simulation device according to any of claims 1 to 9;
ii) A negative pressure wound therapy dressing arranged over at least a portion of the second side of the permeable tissue mimicking construct of the physiological simulation device, to thereby allow negative pressure to be applied to tissue arranged thereon, in use.

11. A method of applying negative pressure wound therapy to *ex vivo* tissue, the method comprising:
i) arranging tissue, such as skin tissue, on a second side of a tissue mimicking construct of a physiological simulation device according to any of claims 1 to 9;
ii) arranging a negative pressure wound therapy dressing over at least a portion of the tissue; and
iii) applying negative pressure wound therapy to the tissue, via the negative pressure wound therapy dressing.

12. A method of monitoring the biological processes of wound healing comprising:
i) arranging tissue having a wounded portion, such as skin tissue having a wounded portion, on a second side of a tissue mimicking construct of a physiological simulation device according to any of claims 1 to 9;
ii) arranging a negative pressure wound therapy dressing over the wounded portion of the tissue;
iii) applying negative pressure wound therapy to the wounded portion of the tissue via the negative pressure wound therapy dressing; and
iv) monitoring the wounded portion of the tissue.

13. A method of applying negative pressure wound therapy to *ex vivo* colonised tissue, said method of applying negative pressure wound therapy to *ex vivo* colonised tissue comprises the steps:
i. colonising tissue, such as skin tissue, with pathogens and/or bacteria;
ii. arranging the colonised tissue on a second side of a tissue mimicking construct of a physiological simulation device according to any of claims 1 to 9;
iii. arranging a negative pressure wound therapy dressing over at least a portion of the colonised tissue; and
iv. applying negative pressure wound therapy to the colonised tissue, via the negative pressure wound therapy dressing.

14. A method of monitoring the biological processes of infection and wound healing, said method of monitoring the biological processes of infection and wound healing comprises the steps:
i. colonising tissue having a wounded portion, such as skin tissue having a wounded portion, with pathogens and/or bacteria;
ii. arranging the colonised tissue having a wounded portion on a second side of a tissue mimicking construct of a physiological simulation device according to any of claims 1 to 9;
iii. arranging a negative pressure wound therapy dressing over the wounded portion of the colonised tissue;
iv. applying negative pressure wound therapy to the wounded portion of the colonised tissue via the negative pressure wound therapy dressing; and
v. monitoring the wounded portion of the colonised tissue.
